Europäisches Patentamt

European Patent Office

Office européen des brevets

(11) Publication number: **0 438 626 A2**

# EUROPEAN PATENT APPLICATION

(21) Application number: 90111059.3

(22) Date of filing: 12.06.90

(51) Int. Cl.5: **A01N 1/00**, A61B 10/00, A61M 1/00

(30) Priority: 26.01.90 US 471084

(43) Date of publication of application:
31.07.91 Bulletin 91/31

(84) Designated Contracting States:
AT BE CH DE DK ES FR GB GR IT LI LU NL SE

(71) Applicant: CRYO-CELL INTERNATIONAL INC.
2300 Long Beach Boulevard
Beach Haven New Jersey 08008(US)

(72) Inventor: Knippscheer, Hermann
2815 Carnation Avenue
Baldwin, New York 11510(US)
Inventor: Richard, Daniel D.
305 Rim Shadows Drive
Sedona, Arizona 86336(US)

(74) Representative: Forattini, Amelia et al
c/o Internazionale Brevetti Ingg. ZINI,
MARANESI & C. S.r.l. Piazza Castello 1
I-20121 Milano(IT)

(54) **Apparatus for preparing an elongate fluid-containing member and related apparatus for removing fluid from such member.**

(57) An apparatus (10) for preparing an elongate fluid-containing member (12) such as an umbilical cord comprises a housing (14) and support elements (30) in the housing for maintaining the umbilical cord in a substantially linear configuration. The support elements are generally horizontally oriented and arranged in a linear array and are pivotably mounted to the housing. A clamping device (42) in the housing applies at least four clamps or staples to the umbilical cord, while a severing device (58) divides the umbilical cord at a point between two midle staples to form at least two separate sections of the umbilical cord. A transfer mechanism in the housing automatically transfers the severed umbilical cord sections to respective receptacles (74), the transfer mechanism including a device (34) operatively connected to the support elements for tilting those elements, the transfer mechanism further including guide funnels (36) disposed in the housing below the support elements for guiding the severed sections of the umbilical cord into the receptacles. A closure assembly (150) is provided for automatically closing the receptacles upon the disposition therein of the severed sections of the umbilical cord. And a control unit (28) is operatively connected to the clamping device, the severing device, the transfer mechanism and the closure assembly for sequencing the operations thereof.

FIG-2

## APPARATUS FOR PREPARING AN ELONGATE FLUID-CONTAINING MEMBER AND RELATED APPARATUS FOR REMOVING FLUID FROM SUCH MEMBER

This invention relates generally to an apparatus for preparing an elongate fluid-containing member for transport and possible storage and eventual utilization. More particularly, this invention relates to an apparatus for packaging an elongate fluid-containing member and/or a part thereof. This invention also relates to an apparatus and a method for removing fluid from a fluid-containing member.

U.S. Patent Application Serial No. 455,170 filed December 22, 1989 and having the same assignee as the instant application discloses in part a method for preparing a biological sample, for ultimate use in an identification process, medical research, or a therapeutic treatment. More particularly, that application discloses a method for preparing the umbilical cord for use in identification, medical research and therapeutic techniques. Even more particularly, that application is directed in part to a method entailing the storage of the umbilical cord, or sections thereof, for future use.

The instant application relates specifically to an apparatus for preparing the umbilical cord, either in sections or in toto, for transport and possible storage and eventual utilization.

As discussed in U.S. Patent Application Serial No. 455,170, the human umbilical cord includes two umbilical arteries and a single umbilical vein. The vein carries oxygenated blood from the placenta to the fetus. The arteries and the vein are embedded in a mucous connective tissue in turn covered by an epithelium which is multilayered at birth. The connective tissue includes interlacing bundles of collagen fibers having intercellular spaces filled with a gelatinous substance known as Wharton's jelly.

The human umbilical cord reaches a length of 35 to 50 cm. However, the cord is typically twisted through several turns and must therefore be held at its ends and stretched to attain its full length.

In many instances, the umbilical cord, like the placenta, is discarded after birth. However, U.S. Patent Application Serial No. 455,170 discloses therapeutic and medical identification uses of umbilical cord segments. To fully appreciate such uses, it is now necessary to provide efficient and reliable means for preparing the umbilical cord segments.

An object of the present invention is to provide an apparatus for preparing an elongate fluid-containing member such as an umbilical cord for transport and possible storage and eventual use.

Another object of the present invention is to provide an apparatus for packaging an elongate fluid-containing member such as an umbilical cord.

A more particular object of the present invention is to provide an apparatus and a method for packaging (preparing for storage) an entire umbilical cord.

Another particular object of the present invention is to provide an apparatus and a method for preparing segments of umbilical cord for transport and/or storage.

Another object of the present invention is to provide such a preparing or packaging apparatus which is efficient and reliable.

A further particular object of the present invention is to provide such a preparing or packaging apparatus which is essentially entirely automatic.

An apparatus for preparing an elongate fluid-containing member, such as an umbilical cord, for transport and/or storage, comprises, in accordance with the present invention, a housing and holding or support elements at least partially in the housing for maintaining the fluid-containing member in a substantially linear configuration. The housing contains a mechanism for applying at least four clamps to the fluid-containing member and a severing device for dividing the fluid-containing member at a point between two middle clamps to form at least two separate sections of the fluid-containing member. A transfer mechanism in the housing automatically transfers the severed sections of the elongate fluid-containing member to respective receptacles.

Pursuant to further features of the present invention, the holding elements support the fluid-containing member in a horizontal orientation and are themselves disposed in a linear array. Preferably, the holding elements have trough-shaped upper support surfaces.

In accordance with a particular embodiment of the present invention, the support elements are pivotably mounted to the housing, the transfer mechanism including pivoting means operatively connected to the support elements for tilting the support elements. In addition, the transfer mechanism includes guides disposed in the housing below the support elements for guiding the severed sections of the fluid-containing member into the receptacles. The guides may take the form of funnels.

Pursuant to yet another feature of the present invention, the transfer mechanism further includes a shifting device in the housing for moving the receptacles into locations juxtaposed to the guide funnels and for subsequently removing the receptacles from those locations upon deposition of the severed sections of the fluid-containing member into the receptacles.

The clamps applied by the clamping mechanism to the fluid-containing member are preferably staples and the clamping mechanism is a stapling device.

Pursuant to a specific feature of the present invention, the holding elements include an additional clamping mechanism or devices attachable to ends of the fluid-containing member for stretching the fluid-containing member.

In accordance with the present invention, the apparatus further comprises a control unit operatively connected to the clamping mechanism, the severing device and the transfer mechanism for sequencing the operations thereof. Advantageously, the apparatus also comprises an information input unit such as a keyboard operatively connected to the control unit (microprocessor) for feeding to the control unit information identifying the fluid-containing member. In addition, an identification device is operatively connected to the control unit for affixing to the receptacles a code designation to identify the contents of the receptacles upon the disposition therein of the severed sections of the fluid-containing member.

Pursuant to yet another feature of the present invention, the apparatus further comprises a closure mechanism for closing the receptacles upon the disposition therein of the severed sections of the fluid-containing member. The closure mechanism preferably includes a screwing device for attaching a screw-type cap to each of the receptacles.

Pursuant to another additional feature of the present invention, the apparatus includes a cleaning system at least partially in the housing for automatically cleaning the fluid-containing member prior to sectioning thereof and for cleaning an interior compartment of the housing upon termination of clamping, severing and transfering operations.

In accordance with a specific embodiment of the present invention, the apparatus further comprises a carriage for shiftably supporting the clamping mechanism and the severing device in the housing. A reciprocatable drive is operatively connected to the carriage for automatically shifting the carriage alternately towards and away from the fluid-containing member while same is held by the holding elements.

Pursuant to yet further features of the present invention, the severing device includes a rotary blade member and a translating mechanism operatively connected to the blade member for shifting the blade member alternately towards and away from the fluid-containing member while same is held by the holding elements.

In accordance with a different embodiment of the present invention, a method for storing an elongate fluid-containing member comprises the steps of (a) clamping or otherwise closing the ends of the fluid-containing member, (b) inserting an end of the fluid-containing member in a recess at the end of a spindle, (c) turning the spindle, (d) winding the fluid-containing member about the spindle during the step of turning, (e) sliding a receptacle over the wound fluid-containing member on the spindle, (f) sliding both the wound fluid-containing member and the receptacle off of the spindle, whereby the wound fluid-containing member remains in a wound state inside the receptacle, and (g) closing the receptacle containing the fluid-containing member.

An apparatus in accordance with the present invention for preparing an elongate fluid-containing member such as an umbilical cord for transport and possible storage and eventual use is automatic, efficient and reliable. The automatic operation and cleaning and sterilization devices serve to eliminate contamination.

In accordance with another aspect of the invention, an apparatus for removing a sample of fluid from a fluid-containing member having an external membrane comprises (a) a frame, (b) a holding element mounted to the frame for supporting the fluid-containing member, and (c) an aspirating device for puncturing the membrane of the fluid-containing member and aspirating a quantity of fluid from the fluid-containing member. A carriage is movably mounted to the frame for movably carrying the aspirating device, while a shifting mechanism is operatively connected to the carriage for shifting the carriage relative to the fluid-containing member on the support or holding element. An actuator is operatively connected to the aspirating device for operating it. The fluid-removing apparatus may also include a mechanism for transporting the removed fluid to another device, such as an automated specimen analysis and identification device.

Fig. 1 is a cross-sectional view, taken in a horizontal plane along line I-I in Fig. 2, of an apparatus for preparing an elongate fluid-containing member, in accordance with the present invention.

Fig. 2 is a cross-sectional view, taken along line II-II in Fig. 1.

Fig. 3 is a side elevational view of a portion of a shifting mechanism for a receptacle support and transfer member shown in Figs. 1 and 2.

Fig. 4 is a partial front elevational view of a transfer mechanism shown in Figs. 1 and 2.

Fig. 5 is a partial top view of a screwing mechanism illustrated in Figs. 1 and 2.

Fig. 6 is a side elevational view, partially in cross-section, of an apparatus for placing an elongate flexible member into a small receptacle.

Fig. 7 is a cross-sectional view, taken along a horizontal plane, of an aspirating apparatus in accordance with the present invention.

Fig. 8 is a vertical cross-sectional view taken along line VIII-VIII in Fig. 7.

Fig. 9 is a vertical cross-sectional view taken along line IX-IX in Fig. 7.

Fig. 10 is a schematic front elevational view of the aspirating apparatus of Figs. 7-9, showing a magazine for loading new hypodermic needles into the apparatus.

Fig. 11 is a schematic diagram of another aspirating apparatus in accordance with the present invention.

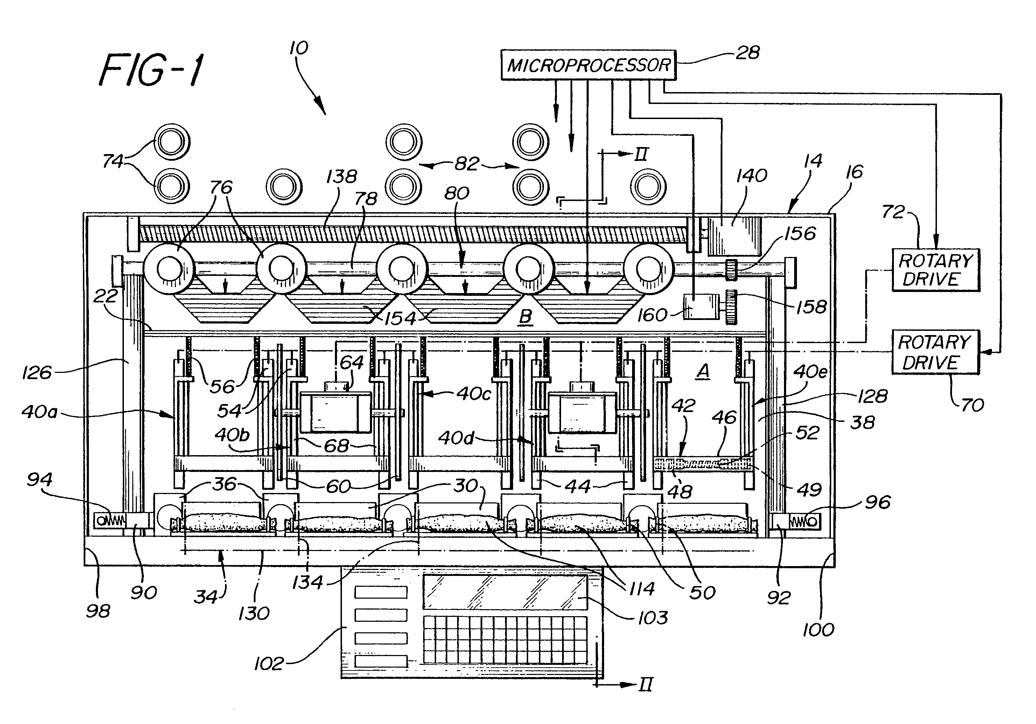

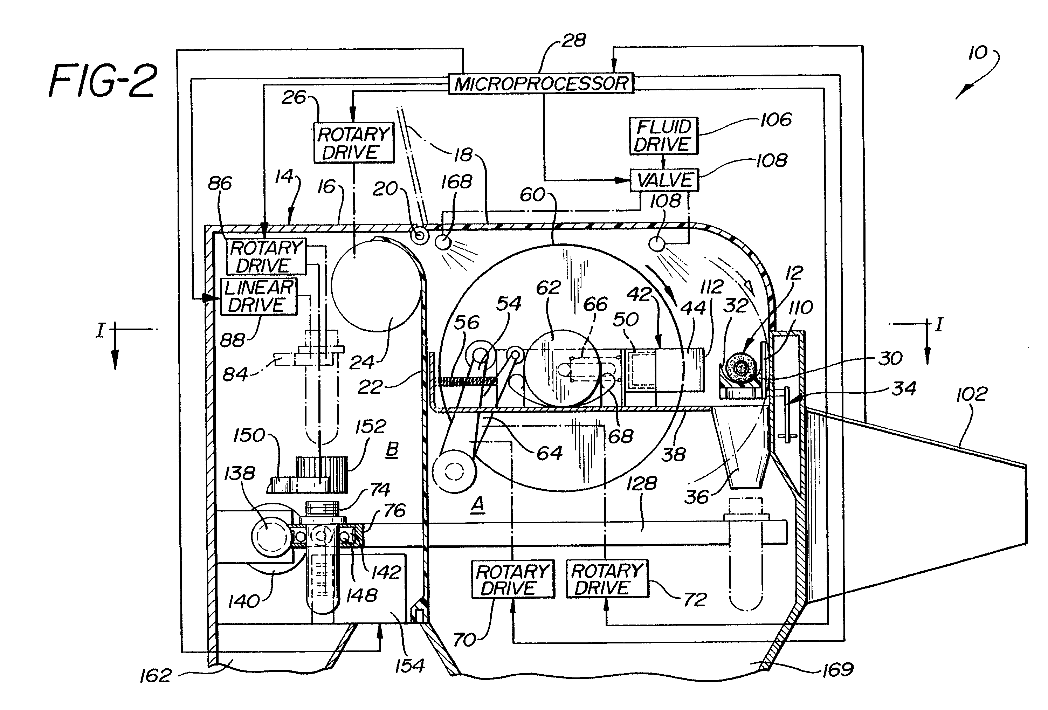

As illustrated in Figs. 1 and 2, an apparatus 10 for preparing an elongate fluid-containing member 12 such as an umbilical cord for transport and possible storage comprises a housing 14 including a body 16 and a hood 18 hingedly secured to body 16 at 20. Housing 14 is subdivided into a first chamber or compartment A and a second chamber or compartment B by a flexible partition member 22 wound at an upper end around a roller 24. Roller 24 is rotatably mounted to body 16 of housing 14 and is operatively connected to a rotary drive 26. Rotary drive 26 is reversible and alternately rotates roller 24 in opposite directions to thereby alternately raise and lower partition member 22, thereby intermittently permitting communication between compartment A and compartment B. Rotary drive 26 is operated in response to signals from a control unit 28 in the form of a microprocessor.

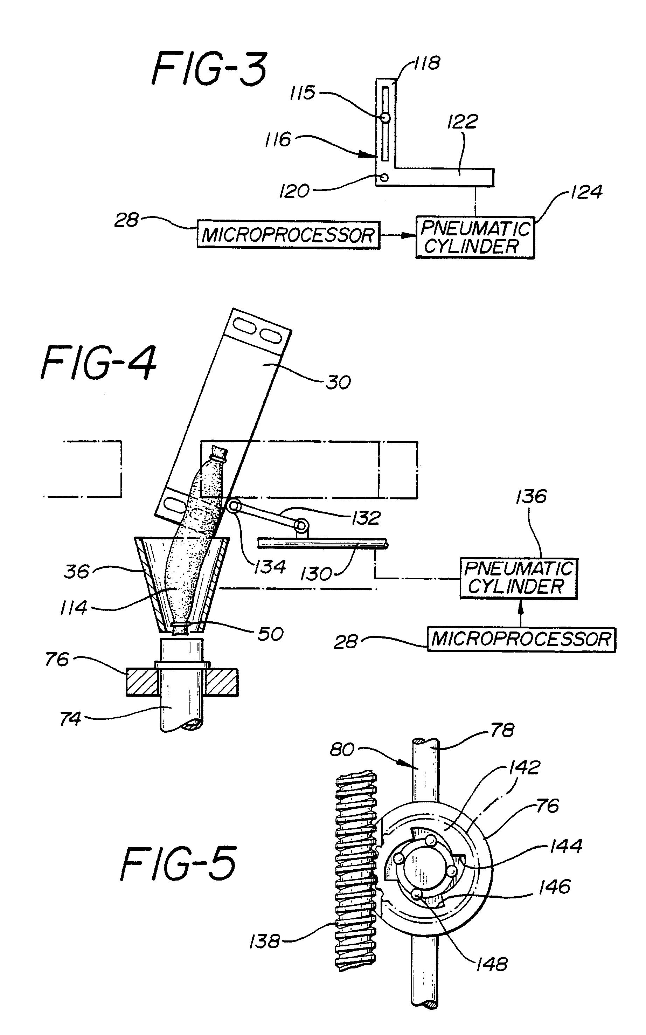

Pivotably mounted to housing body 16 in compartment A of housing 14 is a plurality of molded support elements 30 arranged in a horizontally oriented linear array. Each support element 30 is provided on an upper side with a trough-shaped support surface 32 for receiving umbilical cord 12. Support elements 30 are each connected to a tilting mechanism 34 described in detail hereinafter with reference to Fig. 4.

Disposed between adjacent support elements 30, in a plane below the level of the support elements, are a plurality of guide funnels 36 equal in number to support elements 30. Funnels 36 are attached at their upper ends to a horizontally extending table or platform member 38 fixed to housing body 16.

Platform 38 supports a plurality of carriages 40a, 40b, 40c, 40d, and 40e each carrying a respective clamping mechanism 42 in the form of a stapling device. Each stapling device 42 includes a pair of stapling heads 44 spaced from one another by a distance corresponding to the length of a respective segment or section to be formed from umbilical cord 12. Each stapling device 42 further includes a staple magazine 46 having two stacks 48 and 49 of staples 50 biased towards respective stapling heads 44 by a compression spring 52.

Each carriage 40a, 40b, 40c, 40d, and 40e is shiftable towards support elements 30 by a respective pair of rocker arms 54 in opposition to a restoring force exerted by tension springs 56 connected at one end to the respective carriage and at the other end to housing body 16.

Alternate carriages 40b and 40d carry, in addition to the respective stapling device 42, a severing device 58 including a pair of circular rotary saw blades 60 and a motor 62 for rotating the blades. Cutting blades 60 and motors 62 are shiftably mounted to carriages 40b and 40d so that the motors and the blades can be moved by rocker arms 64 relative to carriages 40b and 40d in opposition to restoring forces exerted by springs 66.

Stapling devices 42 are also shiftably mounted with respect to the respective carriages 40 (including carriages 40a and 40b), restoring springs 68 being provided for biasing the stapling devices in a direction away from support elements 30.

Rocker arms 54 and 64 are operatively linked to respective rotary drives 70 and 72 each in turn operated by microprocessor control unit 28.

Prior to a cutting step in a cycle of operation, as described hereinafter, compartment B contains a plurality of receptacles 74 in the form of ampules or vials each held in a respective collar 76 connected to the other collars by a plurality of rod sections 78 arranged in a linear array. Collars 76 and rod sections 78 are parts of an integrally molded receptacle support and transfer member 80.

Ampules 74 are automatically inserted in collars 76 from stocks 82 by respective robot arm assemblies 84 each including an arm shiftable by virtue of a linear drive 88 and preferably rotatable by virtue of another drive 86. Drives 86 and 88 are operated in response to signals from microprocessor control unit 28.

In a cycle of operation of the apparatus of Figs. 1 and 2 preparing umbilical cord 12 for transfer or storage, hood 18 is opened and cord 12 is grasped at its ends by a pair of clamps 90 and 92 (Fig. 1). Clamps 90 and 92 may be spring loaded as indicated at 94 and 96 or, alternatively, may be connected to respective weights (not shown) by cords hanging over edges 98 and 100 of housing body 16. The weights or the springs 94 and 96 serve to stretch umbilical cord 12 and cause it to maintain a linear form during subsequent stapling and severing operations.

Upon the application of clamps 90 and 92 to the ends of umbilical cord 12 and a disposition of the cord along trough-shaped surfaces 32 of support elements 30, hood 18 is closed. Information regarding the umbilical cord 12 (e.g., name and birth date) is entered into the memory of microprocessor control unit 28 via a keyboard 102 with an integral LCD display window 103. Upon the entry of the requisite information, the microprocessor control unit 28 is instructed via the keyboard to begin processing of the umbilical cord.

First, microprocessor control unit 28 operates a valve 104 or plurality of valves (not shown) to enable the flow of cleansing fluid from a pressurized fluid source 106 through nozzles 108 inside housing compartment A. The spray from nozzles 108 cleans umbilical cord 12.

Upon the lapse of a predetermined period of time programmed into microprocessor control unit 28, the control unit closes valve 104, terminating the cleaning operation, and energizes rotary drives 70 to pivot rocker arms 54 and thereby drive carriages 40a, 40b, 40c, 40d, and 40e towards support elements 30 and umbilical cord 12 supported thereby. The pivoting motion of rocker arms 54 and the concomitant shifting of carriages 40a, 40b, 40c, 40d, and 40e results in a pinning of umbilical cord 12 against a thrust plate 110 by forward edges 112 of carriages 40a, 40b, 40c, 40d, and 40e and in an ejection of staples 50 from stapling heads 44 against the thrust plate, whereby staples 50 are bent around umbilical cord 12 to clamp or pinch it at the locations of stapling heads 44.

Upon the clamping of umbilical cord 12 by staples 50, microprocessor control unit 28 energizes motors 62 to rotate blades 60 and activates rotary drives 72 to pivot rocker arms 64, thereby shifting blades 60 relative to carriages 40b and 40d and into cutting engagement with umbilical cord 12. The umbilical cord 12 is accordingly severed into a plurality of segments 114 each of which is clamped at its ends by a pair of staples 50.

It is to be understood that platform 38, as well as funnels 36 are provided with slots (not illustrated) through which blades 60 pass during the cutting operations.

Upon the severing of umbilical cord 12 by blades 60, microprocessor control unit 28 operates drives 70 and 72 to enable a return of carriages 40a, 40b, 40c, 40d, and 40e, stapling devices 42, blades 60 and motors 62 to their home or rest positions.

After the termination of the cleaning operation and during the clamping and severing operations described above, microprocessor control unit 28 energizes drives 86 and 88 to operate robot arm assemblies 84 to transfer ampules 74 from stocks 82 into respective collars 76. Microprocessor 28 then controls drive 26 to raise partition member 22.

As illustrated in Fig. 3, receptacle support and transfer member 80 is operatively linked via a pin 115 to an arm 118 of a lever arm drive member 116. Member 116 is pivotably connected at 120 to housing body 16 and has a second arm 122 which is connected to a pneumatic cylinder 124 operated under the control of microprocessor 28. Upon the opening of door or partition member 22, microprocessor control unit 28 operates pneumatic cylinder 124 to pivot lever arm drive member 116 and thereby shift receptacle support and transfer member 80 along a pair of rails 126 and 128 from compartment B into compartment A to a position directly below guide funnels 36.

As illustrated in Figs. 1 and 2 and in detail in Fig. 4, tilting mechanism 34 includes a main drive rod 130 connected to a pneumatic cylinder 136 responsive to microprocessor control unit 28. Pivotably connected to main rod 130 at spaced locations therealong is a plurality of linkage elements 132 each in turn fixed at an end opposite main rod 130 to a shaft 134 in turn rigid with a respective support element 30.

Upon the completion of the cutting operation which severs umbilical cord 12 into segments 114, microprocessor control unit 28 operates pneumatic cylinder 136 to longitudinally shift main rod 130 and thereby tilt support elements 30, as illustrated in Fig. 4. The tilting of support elements 30 cause segments 114 to slide from trough-shaped surfaces 32 through guide funnels 36 into ampules 74 at the lower ends of the funnels.

Upon a predetermined period of time allowing for the sliding of umbilical cord segments 114 into ampules 74, microprocessor control unit 28 reverses the operating state of pneumatic cylinder 124 to thereby pivot lever arm drive member 116 back towards its original orientation and concomitantly shift receptacle support and transfer member 80 back along rails 126 and 128 from compartment A into compartment B.

Upon reaching compartment B, partition member 22 is lowered and receptacle support and transfer member 80 is driven by cylinder 124 and lever arm drive member 116 against a screw or worm rod 138 rotatably driven by a motor 140 in response to control signals from microprocessor 28.

As illustrated in Fig. 5, each collar 76 is provided with an internal worm gear 142. An external thread on each worm gear 142 meshes with an external thread on rod 138, so that the turning of rod 138 by motor 140 turns worm gears 142 within collars 76. Worm gears 142 are each provided with a central aperture 144 formed along its periphery with a series of substantially triangular cutouts 146. Ampules 74 traverse apertures 144 and are locked to the rotating worm gears 142 by a plurality of ball bearings 148 which are seated in cutouts 146. Upon the locking of ampules 74 to worm gears 142, the ampules rotate with the worm gears. Microprocessor control unit 28 then operates a robot arm assembly 150 (Fig. 2) to move internally threaded caps 152 from cap stores (not illustrated) onto the externally threaded upper ends of ampules 74. The relative rotation of ampules 74 and caps 152 results in an automatic screwing of caps 152 onto ampules 74.

As described in U.S. Patent Application Serial No. 455,170, caps 152 are advantageously made at least partially of a magnetic material. Specifically, the caps may have synthetic resin bodies provided with metallic inlays. The magnetic material enables automatic handling of the ampules by conveying and transfering devices equipped with electromagnets.

During the screwing of caps 152 onto ampules 74, as described hereinabove, microprocessor control unit 28 transmits information and control signals to a plurality of bar code application units 154. The transmitted information identifies the umbilical cord segments 114 as originating from the same umbilical cord 12. Bar code application units 154 apply circular bar code markings to the outer surface of each ampule during the rotation thereof via worm spindle 138 and worm gears 142. Units 154 may function to attach printed labels to the ampules' outer surfaces or, alternatively, may incorporate laser or other equipment for etching the outer surfaces of the ampules. Other bar code application methods are also within the contemplation of the invention.

Upon the termination of the screwing operation, microprocessor control unit 28 de-energizes motor 140 and actuates pneumatic cylinder 124 to pivot lever arm drive member 116 away from worm spindle 138 and so that a gear 156 on receptacle support and transfer member 80 engages a drive gear 158 rotatably driven by a motor 160. Upon engagement of gears 156 and 158, receptacle support and transfer member 80 rotates to spill the closed segment-containing ampules 74 from collars 76 into a funnel or hopper 162 from which the ampules are conveyed to a long term cryogenic storage apparatus.

After receptacle support and transfer member 80 and its ampules 74 are removed from compartment A and partition member 22 is closed, microprocessor control unit 28 opens valve 104 to enable the flow of cleansing fluid from source 106 through nozzles 108 and 168 inside housing compartment A. The spray from nozzles 108 and 168 cleans compartment A and is drained from the compartment through a funnel 169.

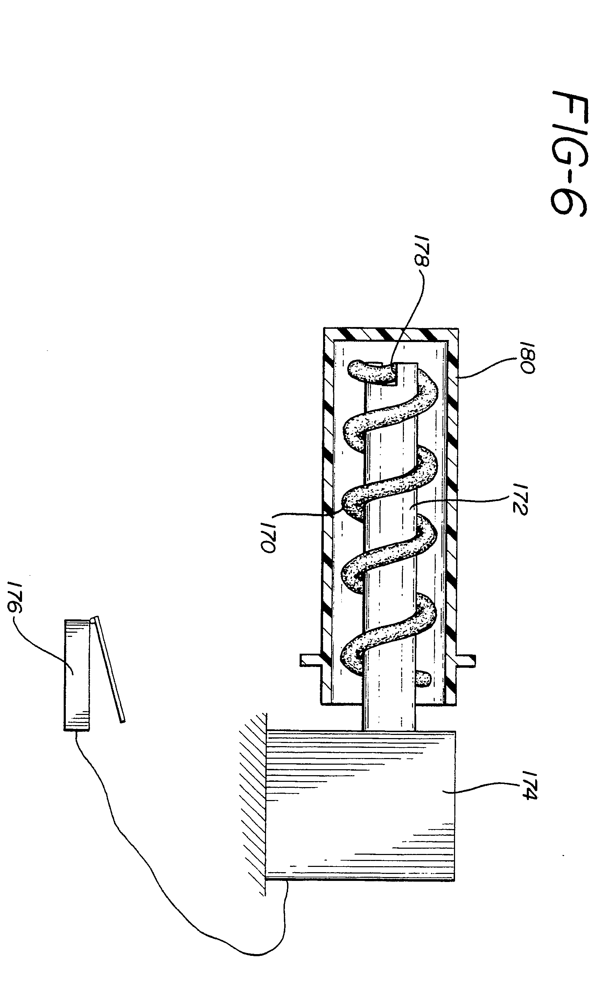

As illustrated in Fig. 6, an assembly for preparing an entire umbilical cord 170 for storage comprises a spindle 172 rotatably mounted in a support 174 which houses a motor (not shown) operatively connected to spindle 172. The motor is energized upon closing a switch or contact 176. Spindle 172 is provided at a free end with a slot or recess 178 into which an end of umbilical cord 170 is inserted.

Upon clamping of the ends of umbilical cord 170, for example by stapling, and upon subsequent insertion of the umbilical cord end into slot 178, switch 176 is closed and then, while spindle 172 is turning, umbilical cord 170 is wound about the spindle. Upon completion of the winding operation, a receptacle 180, exemplarily an ampule, is slid over the wound umbilical cord 170. Subsequently, both the wound umbilical cord 170 and receptacle 180 are removed from the end of spindle 172. A cap (not depicted) is then secured to the open end of receptacle 180, sealing the receptacle. A bar code (not shown) identifying the stored umbilical cord 170 may then be applied to the outer surface of the receptacle.

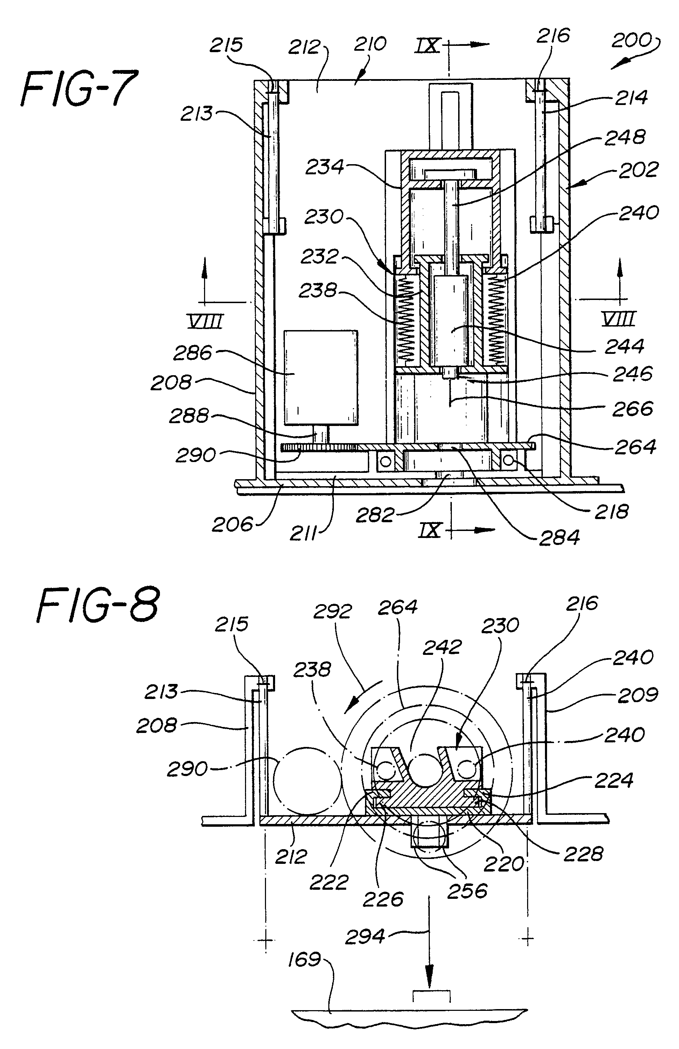

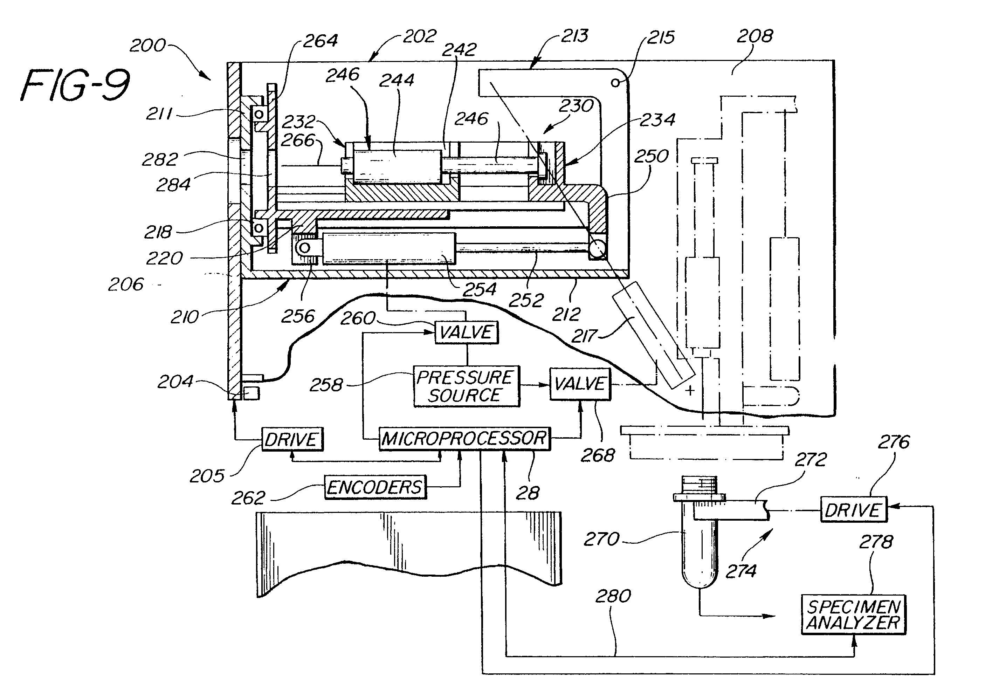

As depicted in Figs. 7-9, an aspirating apparatus 200 for use separately or as an integral part of apparatus 10 of Figs. 1 and 2 comprises a carriage 202 slidably mounted to a plurality of rails 204 (only one rail illustrated, in Fig. 9 of the drawing) and movable along those rails through the action of a drive 205 which is energized in response to control signals from microprocessor control unit 28. Carriage 202 is generally in the shape of a U with a front plate 206 and a pair of side plates 208 and 209.

Swingably mounted to carriage 202 is a rocker member 210 having a front plate 211, a base plate 212 and a pair of L-shaped rocker arms 213 and 214 connected to side plates 208 and 209 at respective pivot points 215 and 216. A pair of pneumatic cylinders 217 (only one shown in Fig. 9) pivotably anchored to side plates 208 and 209 have their plungers connected to L-shaped rocker arms 213 and 214.

Rotatably mounted to front plate 211 of rocker member 210 via an annular bearing 218 is a holder or frame member 220. Holder or frame 220 is provided with a pair of channels 222 and 224 (Fig. 8) which receive respective flanges 226 and 228 of a carrier assembly 230. Carrier assembly 230 includes a forward component 232 and a rearward component 234 slidably connected to one another. Forward and rearward components 232 and 234 are biased by a pair of compression springs 238 and 240 into a spread-apart or extended configuration shown in Figs. 7 and 9. Forward carrier component 232 is provided with an elongate, trough shaped recess or chamber 242, which is open on an upper side for enabling the reception and discharge of the cylindrical hollow body portion 244 of a hypodermic needle assembly 246. Needle assembly 246 also includes a plunger portion 248 entrained to rearward carrier component 234.

As best seen in Fig. 9, rearward carrier component 234 if formed with a downwardly projecting flange 250 connected to the free end of a plunger 252 of a pneumatic cylinder 254. Pneumatic cylinder 254 is connected at an end opposite plunger 252 to a pair of downwardly projecting lugs 256 integral with holder or frame member 220. Cylinder 254 is connectable to a source of pressurized air 258 via a valve 260 operated by microprocessor control unit 28.

Aspirating apparatus 200 is disposed alongside severing and packaging apparatus 10 so that carriage 202 moves in a direction perpendicular to the linear array of support elements 30. Aspirating apparatus is disposed inside housing 14 alongside rail 128.

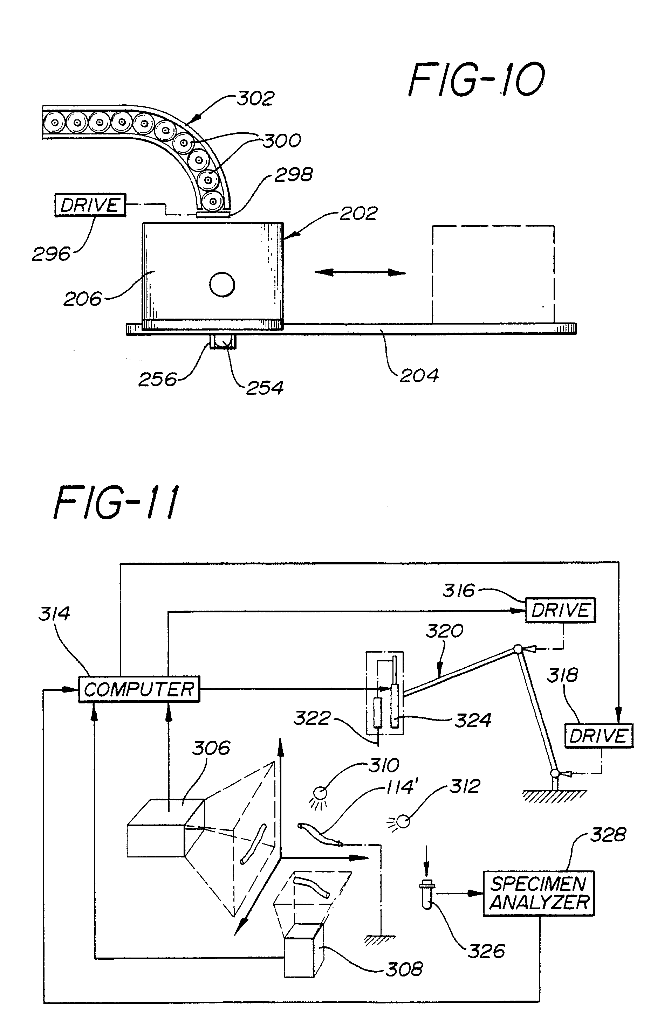

At the onset of an operating cycle of aspirating apparatus 200, microprocessor control unit 28 energizes motor or drive 205 to move carriage 202 from a rest position, shown in solid lines in Fig. 10, to a working position in which the aspirating unit has displaced clamp 92 (Fig. 1) from its location at the end of the linear array of support elements 30. Microprocessor control unit 28 determines that carriage 202 has reached its working position by any one of several different methods. As shown in Fig. 9, microprocessor control unit 28 may receive input from encoders 262, thereby informing microprocessor control unit 28 of the exact position of carriage 202 along rails 204. Alternatively, the arrival of carriage 202 at its working position may be sensed by photoelectric cells (not shown) or other detectors.

Upon the arrival of carriage 202 at its working position, microprocessor control unit 28 stops drive 205 and then opens valve 260 to cause cylinder 254 to retract its plunger 252 and thereby drive rearward carrier component 234 in a forward direction, i.e., towards front plates 206 and 211. During an initial portion of that forward motion, rearward carrier component 234 entrains forward carrier component 232 by pushing against compression springs 238 and 240 which in turn press against forward carrier component 232 and cause it also to move in the forward direction. When forward carrier component 232 comes into contact with a circular toothed plate member or gear 264 at a forward end of frame member 220, rearward carrier component 234 continues to move forward under the action of cylinder 254 and against the restoring forces exerted by compression springs 238 and 240.

Upon the completion of a forward stroke of rearward carrier component 234, detected by microprocessor control unit 28 via encoders 262, a microswitch (not illustrated) or a photoelectric sensor (not shown) or other device, the microprocessor reverses the operation of pneumatic cylinder 254 via valve 260. During an initial part of a return stroke of rearward carrier component 234, forward carrier component 232 remains in a forwardmost position in contact with toothed plate member or gear 264, under the action of compressions springs 238 and 240. It is during that initial part of return stroke of rearward carrier component 234 that fluid is withdrawn or aspirated from the open end of umbilical cord 12. Subsequently, upon the engagement of a front end of rearward carrier component 234 with a back end of forward carrier component 232, the forward carrier component is entrained by the rearward carrier component and also moves in a rearward direction to thereby withdraw needle 266 of assembly 146 from umbilical cord 12.

Upon the completion of the return stroke of rearward carrier component 234, microprocessor control unit 28 opens valve 268, thereby actuating cylinders 217 to retract their plungers and tilt rocker member 210 about pivot points 215 and 216. Rocker member 210, frame member 220, carrier assembly 230 and hypodermic needle assembly 246 are all turned via the action of cylinders 217 from a horizontal, aspirating orientation to a vertical ejection orientation shown in phantom lines in Fig. 9. Upon the attainment of that vertical orientation, microprocessor control unit 28 again operates cylinder 254. During an initial stage of the ejection operation, rearward carrier component 234 and forward carrier component 232 move downwardly together until the tip of needle 266 reaches a vertical position within the mouth of an ampule 270. That position is reached upon the engagement of forward carrier component 232 with toothed plate member 264. During a subsequent stage of the ejection operation, rearward carrier component 234 continues to move under the action of pneumatic cylinder 254, thereby ejecting the fluid contents of cylinder 244 into ampule 270.

Ampule 270 is held by an arm 272 of a conveyance mechanism 274 including a drive 276 operated under the control of microprocessor 28. In response to output signals of microprocessor control unit 28, conveyance mechanism 274 transports ampule 270 to a sample analyzing machine 278. Such a machine may be a computer controlled blood analyzer or a chemoluminescence analyzer with an output lead 280 extending to microprocessor control unit 28 for feeding thereto encoded information regarding the constituents of the fluid sample obtained via aspirating apparatus 200. This encoded information may then be stored in the memory of microprocessor 28, or in another, central computer, together with the information entered via keyboard 102 concerning the identity of the newborn individual.

Upon the completion of the fluid ejection operation, microprocessor control unit 28 again reverses the operation of pneumatic cylinder 254 and causes the withdrawal of plunger 248 from cylinder 244 and the subsequent withdrawal of needle 266 back from the mouth of ampule 268 and through apertures 282 and 284 in front plate 211 and circular toothed plate member 264, respectively. Cylinders 217 are then operated by microprocessor control unit 28 to return rocker member 210 and the components supported thereby to the horizontal, aspirating orientation shown in solid lines in Fig. 9.

As shown in Figs. 7-9, rocker member 210 supports a motor 286 having an output shaft 288 carrying a toothed gear 290 meshingly engaging circular toothed plate member 264. Upon the return of rocker member 210, and the components supported thereby, to the horizontal, aspirating orientation, microprocessor control unit 28 energizes motor 286 to rotate frame member 220 approximately 180°, as indicated by an arrow 292, whereupon hypodermic assembly 246 falls from trough shaped recess 242 into hopper 169, as indicated by an arrow 294 in Fig. 8.

Upon the falling of the used hypodermic needle 246 into hopper or funnel 169, microprocessor control unit 28 reverses motor 286 to return frame member 220, as well as carrier assembly 230, to the initial angular orientation of those elements shown in solid lines in Fig. 8. Microprocessor control unit 28 then activates drive 205 to slide carriage 202 back from the working position (dashed lines in Fig. 10) to the rest or home position (solid lines). Upon the reaching of the home position by carriage 202, a drive 296 temporarily opens a door 298 to enable a new hypodermic needle 300 to fall from a magazine 302 of sterile needles into carrier assembly 230.

After the departure of carriage 202 from the working position juxtaposed to the end of umbilicial cord 12, clamp 92 is attached to the cord end to begin the segmenting process described hereinabove with reference to Figs. 1 and 2.

As illustrated in Fig. 11, in a related method for automatically removing blood or other fluid from an umbilical cord segment 114' held by a support 304, a pair of CCD cameras 306 and 308 scan the segment which is illuminated by light from one or more radiation sources 310 and 312. Cameras 306 and 308 have output signals which are analyzed by a computer 314 in response to programming which exists for optically determining details of an object. Upon determining the location of the vein or an artery in umbilical segment 114', computer 314 activates drives 316 and 318 to move a supporting arm assembly 320 so that an aspirating needle 322 at the free end of assembly 320 is positioned to withdraw fluid from the selected vein or artery. Upon the positioning of the needle, computer 314 operates an actuator 324 to in turn operates needle to aspirate fluid from umbilical cord segment 114'. The computer then activates drives 316 and 318 and actuator 324 to eject the aspirated fluid into a container 326 via which the fluid is delivered to a specimen analyzer 328 as discussed above.

Segment 114' may be obtained from the apparatus 10 described hereinabove. It is to be noted that the video feedback described above with reference to Fig. 11 may be used in combination with the aspirating apparatus of Figs. 7-10 to facilitate the insertion of needle 266 into a fluid bearing component of umbilical cord 12.

It is to be further noted that it is within the contemplation of the invention that the support 304 is movable instead of, or in addition to, moving needle 322. Moreover, aspirating apparatus 200 may also be used in combination with a mechanism for shifting an umbilical cord relative to carriage 202.

A sample of the fluid within an umbilical cord may be removed from the cord by other methods within the contemplation of the present invention. For example, upon a closing (e.g., stapling) of the ends of the umbilical cord, a hollow needle may be inserted through the epithelium of the umbilical cord into the vein thereof. The needle is preferably inserted near the clamp or staple at one end of the cord. Upon the aspiration of a predetermined volume of umbilical cord blood, the needle is withdrawn and a second clamp (e.g., a staple) is placed around the cord on a side of the needle puncture opposite the first staple or clamp. The end portion of the umbilical cord in which the needle puncture was introduced is then severed from the cord and discarded.

Although the invention has been described in terms of particular embodiments and applications, one of ordinary skill in the art, in light of this teaching, can generate additional embodiments and modifications without departing from the spirit of or exceeding the scope of the claimed invention. For example, stapling devices 42 can be mounted on one carriage, rather than several carriages 40a, 40b, 40c, 40d, and 40e. In addition, blades 60 may be separately driven so that umbilical cord segments of different lengths may be produced. Two lever arm guide members as illustrated in Fig. 3 may be provided, one on each side of housing body 16. By way of further example, the identification information corresponding to the umbilical cord segments stored in ampules 74 may be coded in formats and by means other than the bar code format. Moreover, the functions of the various pneumatic drives described herein may be implemented by other mechanisms, e.g., hydraulically or electrically. And the cutting or severing operation may be performed by lasers or other techniques, as alternatives to rotary cutting blades 60. Accordingly, it is to be understood that the drawings and descriptions herein are proferred by way of example to facilitate comprehension of the invention and should not be construed to limit the scope thereof.

Where technical features mentioned in any claim are followed by reference signs, those reference signs have been included for the sole purpose of increasing the intelligibility of the claims and accordingly, such reference signs do not have any limiting effect on the scope of each element identified by way of example by such reference signs.

## Claims

1. Apparatus for preparing an elongate fluid-containing member (12), characterized in that it comprises:
   holding means (30) for maintaining the fluid-containing member (12) in a substantially linear configuration;
   clamping means (42) for applying at least four clamps (50) to said fluid-containing member while said fluid-containing member is being held in a substantially linear configuration by said holding means; and
   severing means (58) for dividing said fluid-containing member at a point between two middle ones of said clamps to form at least two separate sections (114) of said fluid-containing member.

2. The apparatus according to claim 1, further comprising a housing (14), said holding means (30) being disposed at least partially in said housing and said clamping means being disposed in said housing.

3. The apparatus according to claim 2, further comprising transfer means (34) in said housing for automatically transferring said sections to respective receptacles (74).

4. The apparatus according to claim 3 wherein said holding means includes a plurality of support elements (30) disposed in a linear array, said support elements being pivotably mounted to said housing, said transfer means including pivoting means (34) operatively connected to said support elements for tilting same.

5. The apparatus according to claim 4 wherein said transfer means further includes guide means (36) disposed in said housing below said support elements for guiding the severed sections (114) of said fluid-containing members into said receptacles (74).

6. The apparatus according to claim 5 wherein said transfer means further includes shifting means (84) in said housing for moving said receptacles (74) into locations (76) juxtaposed to said guide means and for subsequently removing said receptacles from said locations upon deposition of the severed sections (114) of said fluid-containing member into said receptacles (74).

7. The apparatus according to claim 3, further comprising identification means (154) for affixing to said receptacles a code designation to identify the contents of said receptacles upon the disposition therein of the severed sections of said fluid-containing member.

8. The apparatus according to claim 3, further comprising closure means (150) for closing said receptacles upon the disposition therein of the severed sections of said fluid-containing member.

9. The apparatus according to claim 3, further comprising control means (28) operatively connected to said clamping means, said severing means and said transfer means for sequencing the operations thereof.

10. The apparatus according to claim 2, further comprising cleaning means (106, 108) at least partially in said housing for automatically cleaning an interior compartment of said housing.

11. The apparatus according to claim 1 wherein said severing means includes a rotary blade member (60).

12. The apparatus according to claims 11, further comprising means 64 operatively connected to said blade member for shifting said blade member alternately towards and away from said fluid-containing member while same is held by said holding means.

13. The apparatus according to claim 1, further comprising means (200) for removing fluid from said fluid-containing member.

14. A method for storing an elongate fluid-containing member, characterized in that it comprises:
    closing both ends of the fluid-containing member (12);
    removably attaching an end of said fluid containing member to an end of a spindle (172);
    turning said spindle;
    winding said fluid-containing member about said spindle during execution of said step of turning;
    sliding a receptacle (180) over the wound fluid-containing member on said spindle;

sliding both the wound fluid-containing member and the receptacle off of the spindle, whereby the wound fluid-containing member remains in a wound state inside the receptacle; and

closing the receptacle containing the fluid-containing member.

15. The apparatus for removing a sample of fluid from a fluid-containing member having an external membrane, characterized in that it comprises:

holding means (30) for supporting the fluid-containing member (12);

aspirating means (200) for puncturing the membrane of said fluid-containing member and aspirating a quantity of fluid from said fluid-containing member while said fluid containing member is supported by said holding means:

carriage means (202) for moving one of said holding means and said aspirating means relative to the other;

shifting means (210) operatively connected to said carriage means for shifting same; and

actuator means operatively connected to said aspirating means for operating same.

16. The apparatus according to claim 15, further comprising means for transporting the removed fluid to another device.

FIG-1

FIG-2

FIG-3

118

115

116

122

120

28 MICROPROCESSOR → PNEUMATIC CYLINDER 124

FIG-4

30

132

36

134 130

114

50

PNEUMATIC CYLINDER 136

76

28 MICROPROCESSOR

74

78

80

142

76

144

FIG-5

146

138

148

FIG-6

FIG-7

FIG-8

FIG-9

EP 0 438 626 A2

16

*FIG-10*

*FIG-11*